# EUROPEAN PATENT APPLICATION

(11) **EP 1 081 226 A1**
(43) Date of publication of application: **07.03.2001**
(21) Application number: 99202858.9
(22) Date of filing: 02.09.1999
(51) Int. Cl.: C12N 15/12, C12N 5/10, C12N 1/19, C07K 14/47, C07K 16/18, A61K 38/17, A61K 31/713, A61P 35/00, A61P 37/00

(54) **Apoptin-associating protein**

(71) Applicant: Leadd B.V., 2333 AL Leiden (NL)
(72) Inventor: The designation of the inventor has not yet been filed
(74) Representative: Ottevangers, Sietse Ulbe

(57) **Abstract**

The invention relates to the field of apoptosis. The invention provides novel therapeutic possibilities, for example novel combinatorial therapies or novel therapeutic compounds that can work alone, sequentially to, or jointly with apoptin, especially in those cases wherein p53 is (partly) non-functional.

### Annex to the application documents - subsequently filed sequences listing

SEQUENCE LISTING

<110> Leadd B.V.

<120> Apoptin-associating protein

<130> P50664EP00

<140> 99202858.9
<141> 1999-09-02

<160> 18

<170> PatentIn Ver. 2.1

<210> 1
<211> 17
<212> DNA
<213> Artificial Sequence

<220>
<223> Description of Artificial Sequence: primer

<220>
<221> primer_bind
<222> (1)..(17)
<223> /note="Primer pACT-specific"

<400> 1
taccactaca atggatg                                                17

<210> 2
<211> 10
<212> PRT
<213> Artificial Sequence

<220>
<223> Description of Artificial Sequence: Myc-tag

<220>
<221> BINDING
<222> (1)..(10)
<223> /note="Myc-tag"

<400> 2
Glu Gln Lys Leu Ile Ser Glu Glu Asp Leu
  1               5                  10

<210> 3
<211> 16
<212> PRT
<213> Homo sapiens

<220>
<221> CHAIN
<222> (1)..(16)
<223> /note="Immunogen"

<400> 3
Cys Thr Lys Thr Ser Glu Thr Asn His Thr Ser Arg Pro Arg Leu Lys
1               5                   10                  15

<210> 4
<211> 947
<212> DNA
<213> Homo sapiens

<220>
<223> Description of Combined DNA/RNA Molecule: apoptin-associating protein

<220>
<221> CDS
<222> (54)..(920)
<223> /note="Apoptin-associating protein"

<400> 4
accanaccca aaaaaagaga tctggaattc ggatcctcga ggccacgaag gcc gaa    56
                                                          Glu
                                                          1

aca gtg ctg aag cct tta aat gca gca tct gcg atg tga gga aag gca    104
Thr Val Leu Lys Pro Leu Asn Ala Ala Ser Ala Met     Gly Lys Ala
            5                   10                  15

cct cca cca gaa aac ctc gga tca att ctc agc tgg tgg cnc aac aag    152
Pro Pro Pro Glu Asn Leu Gly Ser Ile Leu Ser Trp Trp Xaa Asn Lys
        20                  25                  30

tgg cac aac agt atg cca ccc cac cac ccc cta aaa agg aga aga agg    200
Trp His Asn Ser Met Pro Pro His His Pro Leu Lys Arg Arg Arg Arg
    35                  40                  45

aga aag ttg aaa agc agg aca aag aga aac ctg aga aag aca agg aaa    248
Arg Lys Leu Lys Ser Arg Thr Lys Arg Asn Leu Arg Lys Thr Arg Lys
50                  55                  60                  65

tta gtc cta gtg tta cca aga aaa ata cca aca aga aaa cca aac caa    296
Leu Val Leu Val Leu Pro Arg Lys Ile Pro Thr Arg Lys Pro Asn Gln
                70                  75                  80

agt ctg aca ttc tga aag atc ctc cta gtg aag caa aca gca tac agt    344
Ser Leu Thr Phe     Lys Ile Leu Leu Val Lys Gln Thr Ala Tyr Ser
            85                  90                  95

ctg caa atg cta caa caa aga cca gcg aaa caa atc aca cct caa ggc    392
Leu Gln Met Leu Gln Gln Arg Pro Ala Lys Gln Ile Thr Pro Gln Gly
        100                 105                 110

ccc ggc tga aaa acg tgg aca gga gca ctg cac agc agt tgg cag taa    440
Pro Gly     Lys Thr Trp Thr Gly Ala Leu His Ser Ser Trp Gln
    115                 120                 125

ctg tgg gca acg tca ccg tca tta tca cag act tta agg aaa aga ctc    488
Leu Trp Ala Thr Ser Pro Ser Leu Ser Gln Thr Leu Arg Lys Arg Leu
130                 135                 140                 145

gct cct cat cga cat cct cat cca cag tga cct cca gtg cag ggt cag    536

Ala Pro His Arg His Pro His Pro Gln     Pro Pro Val Gln Gly Gln
                150                 155                 160

aac agc aga acc aga sca gct cgg ggt cag aga gca cag aca agg gct    584
Asn Ser Arg Thr Arg Xaa Ala Arg Gly Gln Arg Ala Gln Thr Arg Ala
            165                 170                 175

cct ccc gtt cct cca cgc caa agg gcg aca tgt cag cag tca atg atg    632
Pro Pro Val Pro Pro Arg Gln Arg Ala Thr Cys Gln Gln Ser Met Met
        180                 185                 190

aat ctt tct gaa att gca cat gga att gtg aaa act atg aat cag ggt    680
Asn Leu Ser Glu Ile Ala His Gly Ile Val Lys Thr Met Asn Gln Gly
    195                 200                 205

atg aaa ttc aaa acc tcc acc tgc cca tgc tgc ttg cat ccc tgg aga    728
Met Lys Phe Lys Thr Ser Thr Cys Pro Cys Cys Leu His Pro Trp Arg
210                 215                 220                 225

atc ttc tgt gga cat cga cct ctt agt gat gct gcc agg ata att tct    776
Ile Phe Cys Gly His Arg Pro Leu Ser Asp Ala Ala Arg Ile Ile Ser
                230                 235                 240

gct tgc cat ggg cat ctg gcc acc aag gaa ttt cgc acc ctg acg att    824
Ala Cys His Gly His Leu Ala Thr Lys Glu Phe Arg Thr Leu Thr Ile
            245                 250                 255

act ctt gac act ttt atg tat tcc att gtt tta tat gat ttt cct aac    872
Thr Leu Asp Thr Phe Met Tyr Ser Ile Val Leu Tyr Asp Phe Pro Asn
        260                 265                 270

aat cat tta taa ttg gat gtg ctc ctg aat cta ctt ttt ata aaa aaa    920
Asn His Leu     Leu Asp Val Leu Leu Asn Leu Leu Phe Ile Lys Lys
    275                 280                 285

gccttygtgg cctcgagaga tctatga                                      947


<210> 5
<211> 13
<212> PRT
<213> Homo sapiens
<223> Description of Combined DNA/RNA Molecule: apoptin-associating protein

<400> 5
Glu Thr Val Leu Lys Pro Leu Asn Ala Ala Ser Ala Met
  1               5                  10



<210> 6
<211> 71
<212> PRT
<213> Homo sapiens
<223> Description of Combined DNA/RNA Molecule: apoptin-associating protein

<400> 6
Gly Lys Ala Pro Pro Pro Glu Asn Leu Gly Ser Ile Leu Ser Trp Trp
  1               5                  10                  15

Xaa Asn Lys Trp His Asn Ser Met Pro Pro His His Pro Leu Lys Arg
             20                  25                  30

Arg Arg Arg Arg Lys Leu Lys Ser Arg Thr Lys Arg Asn Leu Arg Lys
        35                  40                  45

Thr Arg Lys Leu Val Leu Val Leu Pro Arg Lys Ile Pro Thr Arg Lys
    50                  55                  60

Pro Asn Gln Ser Leu Thr Phe
65                  70

<210> 7
<211> 29
<212> PRT
<213> Homo sapiens
<223> Description of Combined DNA/RNA Molecule: apoptin-associating protein

<400> 7
Lys Ile Leu Leu Val Lys Gln Thr Ala Tyr Ser Leu Gln Met Leu Gln
  1               5                  10                  15

Gln Arg Pro Ala Lys Gln Ile Thr Pro Gln Gly Pro Gly
            20                  25

<210> 8
<211> 12
<212> PRT
<213> Homo sapiens
<223> Description of Combined DNA/RNA Molecule: apoptin-associating protein

<400> 8
Lys Thr Trp Thr Gly Ala Leu His Ser Ser Trp Gln
  1               5                  10

<210> 9
<211> 25
<212> PRT
<213> Homo sapiens
<223> Description of Combined DNA/RNA Molecule: apoptin-associating protein

<400> 9
Leu Trp Ala Thr Ser Pro Ser Leu Ser Gln Thr Leu Arg Lys Arg Leu
  1               5                  10                  15

Ala Pro His Arg His Pro His Pro Gln
            20                  25

<210> 10
<211> 121
<212> PRT
<213> Homo sapiens
<223> Description of Combined DNA/RNA Molecule: apoptin-associating protein

<400> 10
Pro Pro Val Gln Gly Gln Asn Ser Arg Thr Arg Xaa Ala Arg Gly Gln
  1               5                  10                  15

Arg Ala Gln Thr Arg Ala Pro Pro Val Pro Pro Arg Gln Arg Ala Thr

20                   25                   30

Cys Gln Gln Ser Met Met Asn Leu Ser Glu Ile Ala His Gly Ile Val
        35                  40                  45

Lys Thr Met Asn Gln Gly Met Lys Phe Lys Thr Ser Thr Cys Pro Cys
    50                  55                  60

Cys Leu His Pro Trp Arg Ile Phe Cys Gly His Arg Pro Leu Ser Asp
65                  70                  75                  80

Ala Ala Arg Ile Ile Ser Ala Cys His Gly His Leu Ala Thr Lys Glu
                85                  90                  95

Phe Arg Thr Leu Thr Ile Thr Leu Asp Thr Phe Met Tyr Ser Ile Val
            100                 105                 110

Leu Tyr Asp Phe Pro Asn Asn His Leu
        115                 120

<210> 11
<211> 12
<212> PRT
<213> Homo sapiens
<223> Description of Combined DNA/RNA Molecule: apoptin-associating protein

<400> 11
Leu Asp Val Leu Leu Asn Leu Leu Phe Ile Lys Lys
  1               5                  10

<210> 12
<211> 1131
<212> DNA
<213> Homo sapiens

<220>
<223> Description of Combined DNA/RNA Molecule: apoptin-associating protein

<220>
<221> CDS
<222> (1)..(1131)
<223> /note="Apoptin-associating protein"

<400> 12
tat aac tat cta ttc gat gat gaa gat acc cca cca aac cca aaa aaa    48
Tyr Asn Tyr Leu Phe Asp Asp Glu Asp Thr Pro Pro Asn Pro Lys Lys
  1               5                  10                  15

gag atc tgg aat tcg gat cct cga ggc cac gaa ggc ctt tct cct ccg    96
Glu Ile Trp Asn Ser Asp Pro Arg Gly His Glu Gly Leu Ser Pro Pro
             20                  25                  30

agc ggc gcc ggt ttc ggc ttg ggg ggg gcg ggg tac agc cca tcc atg    144
Ser Gly Ala Gly Phe Gly Leu Gly Gly Ala Gly Tyr Ser Pro Ser Met
         35                  40                  45

acc atg ggc gac aag aag agc ccg acc agg cca aaa aga caa gcg aaa    192
Thr Met Gly Asp Lys Lys Ser Pro Thr Arg Pro Lys Arg Gln Ala Lys

    50                  55                  60

cct gcc gca gac gaa ggg ttt tgg gat tgt agc gtc tgc acc ttc aga 240
Pro Ala Ala Asp Glu Gly Phe Trp Asp Cys Ser Val Cys Thr Phe Arg
65                  70                  75                  80

aac agt gct gaa gcc ttt aaa tgc agc atc tgc gat gtg agg aaa ggc 288
Asn Ser Ala Glu Ala Phe Lys Cys Ser Ile Cys Asp Val Arg Lys Gly
                85                  90                  95

acc tcc acc aga aaa cct cgg atc aat tct cag ctg gtg gca caa caa 336
Thr Ser Thr Arg Lys Pro Arg Ile Asn Ser Gln Leu Val Ala Gln Gln
            100                 105                 110

gtg gca caa cag tat gcc acc cca cca ccc cct aaa aag gag aag aag 384
Val Ala Gln Gln Tyr Ala Thr Pro Pro Pro Pro Lys Lys Glu Lys Lys
        115                 120                 125

gag aaa gtt gaa aag cag gac aaa gag aaa cct gag aaa gac aag gaa 432
Glu Lys Val Glu Lys Gln Asp Lys Glu Lys Pro Glu Lys Asp Lys Glu
    130                 135                 140

att agt cct agt gtt acc aag aaa aat acc aac aag aaa acc aaa cca 480
Ile Ser Pro Ser Val Thr Lys Lys Asn Thr Asn Lys Lys Thr Lys Pro
145                 150                 155                 160

aag tct gac att ctg aaa gat cct cct agt gaa gca aac agc ata cag 528
Lys Ser Asp Ile Leu Lys Asp Pro Pro Ser Glu Ala Asn Ser Ile Gln
                165                 170                 175

tct gca aat gct aca aca aag acc agc gaa aca aat cac acc tca agg 576
Ser Ala Asn Ala Thr Thr Lys Thr Ser Glu Thr Asn His Thr Ser Arg
            180                 185                 190

ccc cgg ctg aaa aac gtg gac agg agc act gca cag cag ttg gca gta 624
Pro Arg Leu Lys Asn Val Asp Arg Ser Thr Ala Gln Gln Leu Ala Val
        195                 200                 205

act gtg ggc aac gtc acc gtc att atc aca gac ttt aag gaa aag act 672
Thr Val Gly Asn Val Thr Val Ile Ile Thr Asp Phe Lys Glu Lys Thr
    210                 215                 220

cgc tcc tca tcg aca tcc tca tcc aca gtg acc tcc agt gca ggg tca 720
Arg Ser Ser Ser Thr Ser Ser Ser Thr Val Thr Ser Ser Ala Gly Ser
225                 230                 235                 240

gaa cag cag aac cag agc agc tcg ggg tca gag agc aca gac aag ggc 768
Glu Gln Gln Asn Gln Ser Ser Ser Gly Ser Glu Ser Thr Asp Lys Gly
                245                 250                 255

tcc tcc cgt tcc tcc acg cca aag ggc gac atg tca gca gtc aat gat 816
Ser Ser Arg Ser Ser Thr Pro Lys Gly Asp Met Ser Ala Val Asn Asp
            260                 265                 270

gaa tct ttc tga aat tgc aca tgg aat tgt gaa aac tat gaa tca ggg 864
Glu Ser Phe Asn Cys Thr Trp Asn Cys Glu Asn Tyr Glu Ser Gly
        275                 280                 285

tat gaa att caa aac ctc cac ctg ccc atg ctg ctt gca tcc ctg gag 912
Tyr Glu Ile Gln Asn Leu His Leu Pro Met Leu Leu Ala Ser Leu Glu
        290                 295                 300

aat ctt ctg tgg aca tcg acc tct tag tga tgc tgc cag gat aat ttc    960
Asn Leu Leu Trp Thr Ser Thr Ser         Cys Cys Gln Asp Asn Phe
305                 310                 315                 320

tgc ttg cca tgg gca tct ggc cac caa gga att tcg cac cct gac gat    1008
Cys Leu Pro Trp Ala Ser Gly His Gln Gly Ile Ser His Pro Asp Asp
                325                 330                 335

tac tct tga cac ttt tat gta ttc cat tgt ttt ata tga ttt tcc taa    1056
Tyr Ser     His Phe Tyr Val Phe His Cys Phe Ile     Phe Ser
            340                 345                 350

caa tca ttt ata att gga tgt gct cct gaa tct act ttt tat aaa aag    1104
Gln Ser Phe Ile Ile Gly Cys Ala Pro Glu Ser Thr Phe Tyr Lys Lys
        355                 360                 365

gcc ttc gtg gcc tcg aga gat cta tga                                1131
Ala Phe Val Ala Ser Arg Asp Leu
    370                 375

<210> 13
<211> 275
<212> PRT
<213> Homo sapiens
<223> Description of Combined DNA/RNA Molecule: apoptin-associating protein

<400> 13
Tyr Asn Tyr Leu Phe Asp Asp Glu Asp Thr Pro Pro Asn Pro Lys Lys
  1               5                  10                  15

Glu Ile Trp Asn Ser Asp Pro Arg Gly His Glu Gly Leu Ser Pro Pro
             20                  25                  30

Ser Gly Ala Gly Phe Gly Leu Gly Gly Ala Gly Tyr Ser Pro Ser Met
         35                  40                  45

Thr Met Gly Asp Lys Lys Ser Pro Thr Arg Pro Lys Arg Gln Ala Lys
     50                  55                  60

Pro Ala Ala Asp Glu Gly Phe Trp Asp Cys Ser Val Cys Thr Phe Arg
 65                  70                  75                  80

Asn Ser Ala Glu Ala Phe Lys Cys Ser Ile Cys Asp Val Arg Lys Gly
                 85                  90                  95

Thr Ser Thr Arg Lys Pro Arg Ile Asn Ser Gln Leu Val Ala Gln Gln
            100                 105                 110

Val Ala Gln Gln Tyr Ala Thr Pro Pro Pro Pro Lys Lys Glu Lys Lys
        115                 120                 125

Glu Lys Val Glu Lys Gln Asp Lys Glu Lys Pro Glu Lys Asp Lys Glu
    130                 135                 140

Ile Ser Pro Ser Val Thr Lys Lys Asn Thr Asn Lys Lys Thr Lys Pro
145                 150                 155                 160

Lys Ser Asp Ile Leu Lys Asp Pro Pro Ser Glu Ala Asn Ser Ile Gln
                165                 170                 175

Ser Ala Asn Ala Thr Thr Lys Thr Ser Glu Thr Asn His Thr Ser Arg
            180                 185                 190

Pro Arg Leu Lys Asn Val Asp Arg Ser Thr Ala Gln Gln Leu Ala Val
        195                 200                 205

Thr Val Gly Asn Val Thr Val Ile Ile Thr Asp Phe Lys Glu Lys Thr
    210                 215                 220

Arg Ser Ser Ser Thr Ser Ser Ser Thr Val Thr Ser Ser Ala Gly Ser
225                 230                 235                 240

Glu Gln Gln Asn Gln Ser Ser Ser Gly Ser Glu Ser Thr Asp Lys Gly
                245                 250                 255

Ser Ser Arg Ser Ser Thr Pro Lys Gly Asp Met Ser Ala Val Asn Asp
            260                 265                 270

Glu Ser Phe
        275

<210> 14
<211> 36
<212> PRT
<213> Homo sapiens
<223> Description of Combined DNA/RNA Molecule: apoptin-associating protein

<400> 14
Asn Cys Thr Trp Asn Cys Glu Asn Tyr Glu Ser Gly Tyr Glu Ile Gln
  1               5                  10                  15

Asn Leu His Leu Pro Met Leu Leu Ala Ser Leu Glu Asn Leu Leu Trp
             20                  25                  30

Thr Ser Thr Ser
         35

<210> 15
<211> 24
<212> PRT
<213> Homo sapiens
<223> Description of Combined DNA/RNA Molecule: apoptin-associating protein

<400> 15
Cys Cys Gln Asp Asn Phe Cys Leu Pro Trp Ala Ser Gly His Gln Gly
  1               5                  10                  15

Ile Ser His Pro Asp Asp Tyr Ser
             20

<210> 16
<211> 9
<212> PRT
<213> Homo sapiens
<223> Description of Combined DNA/RNA Molecule: apoptin-associating protein

<400> 16

His Phe Tyr Val Phe His Cys Phe Ile
1               5

<210> 17
<211> 24
<212> PRT
<213> Homo sapiens
<223> Description of Combined DNA/RNA Molecule: apoptin-associating protein

<400> 17
Gln Ser Phe Ile Ile Gly Cys Ala Pro Glu Ser Thr Phe Tyr Lys Lys
1               5                   10                  15

Ala Phe Val Ala Ser Arg Asp Leu
            20

<210> 18
<211> 352
<212> PRT
<213> Homo sapiens

<220>
<221> CHAIN
<222> (1)..(352)
<223> /note="Apoptin-associating protein"

<400> 18
His Glu Gly Leu Ser Pro Pro Ser Gly Ala Gly Phe Gly Leu Gly Gly
1               5                   10                  15

Ala Gly Tyr Ser Pro Ser Met Thr Met Gly Asp Lys Lys Ser Pro Thr
            20                  25                  30

Arg Pro Lys Arg Gln Ala Lys Pro Ala Ala Asp Glu Gly Phe Trp Asp
        35                  40                  45

Cys Ser Val Cys Thr Phe Arg Asn Ser Ala Glu Ala Phe Lys Cys Ser
    50                  55                  60

Ile Cys Asp Val Arg Lys Gly Thr Ser Thr Arg Lys Pro Arg Ile Asn
65                  70                  75                  80

Ser Gln Leu Val Ala Gln Gln Val Ala Gln Gln Tyr Ala Thr Pro Pro
                85                  90                  95

Pro Pro Lys Lys Glu Lys Lys Glu Lys Val Glu Lys Gln Asp Lys Glu
            100                 105                 110

Lys Pro Glu Lys Asp Lys Glu Ile Ser Pro Ser Val Thr Lys Lys Asn
        115                 120                 125

Thr Asn Lys Lys Thr Lys Pro Lys Ser Asp Ile Leu Lys Asp Pro Pro
    130                 135                 140

Ser Glu Ala Asn Ser Ile Gln Ser Ala Asn Ala Thr Thr Lys Thr Ser
145                 150                 155                 160

Glu Thr Asn His Thr Ser Arg Pro Arg Leu Lys Asn Val Asp Arg Ser

                165                 170                 175

Thr Ala Gln Gln Leu Ala Val Thr Val Gly Asn Val Thr Val Ile Ile
            180                 185                 190

Thr Asp Phe Lys Glu Lys Thr Arg Ser Ser Ser Thr Ser Ser Ser Thr
        195                 200                 205

Val Thr Ser Ser Ala Gly Ser Glu Gln Gln Asn Gln Ser Ser Ser Gly
    210                 215                 220

Ser Glu Ser Thr Asp Lys Gly Ser Ser Arg Ser Ser Thr Pro Lys Gly
225                 230                 235                 240

Asp Met Ser Ala Val Asn Asp Glu Ser Phe Xaa Asn Cys Thr Trp Asn
                245                 250                 255

Cys Glu Asn Tyr Glu Ser Gly Tyr Glu Ile Gln Asn Leu His Leu Pro
            260                 265                 270

Met Leu Leu Ala Ser Leu Glu Asn Leu Leu Trp Thr Ser Thr Ser Xaa
        275                 280                 285

Xaa Cys Cys Gln Asp Asn Phe Cys Leu Pro Trp Ala Ser Gly His Gln
    290                 295                 300

Gly Ile Ser His Pro Asp Asp Tyr Ser Xaa His Phe Tyr Val Phe His
305                 310                 315                 320

Cys Phe Ile Xaa Phe Ser Xaa Gln Ser Phe Ile Ile Gly Cys Ala Pro
                325                 330                 335

Glu Ser Thr Phe Tyr Lys Lys Ala Phe Val Ala Ser Arg Asp Leu Xaa
            340                 345                 350

## Description

The invention relates to the field of apoptosis. Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).

The apoptotic process can be initiated by a variety of regulatory stimuli (Wyllie, 1995, White 1996, Levine, 1997). Changes in the cell survival rate play an important role in human pathogenesis of diseases, e.g. in cancer development and auto-immune diseases, where enhanced proliferation or decreased cell death (Kerr et al., 1994, Paulovich, 1997) is observed. A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Certain transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).

For such tumors lacking functional p53 (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or can not be blocked by anti-apoptotic activities, such as Bcl-2 or Bcr-abl-like ones. These factors might be part of a distinct apoptosis pathway or might be (far) downstream of the apoptosis inhibiting compounds.

Apoptin is a small protein derived from chicken anemia virus (CAV; Noteborn and De Boer, 1995, Noteborn et al., 1991, Noteborn et al., 1994; 1998a), which can induce apoptosis in human malignant and transformed cell lines, but not in untransformed human cell cultures. In vitro, apoptin fails to induce programmed cell death in normal lymphoid, dermal, epidermal, endothelial and smooth-muscle cells. However, when normal cells are transformed they become susceptible to apoptosis by apoptin. Long-term expression of apoptin in normal human fibroblasts revealed that apoptin has no toxic or transforming activity in these cells (Danen-van Oorschot, 1997 and Noteborn, 1996).

In normal cells, apoptin was found predominantly in the cytoplasm, whereas in transformed or malignant cells i.e. characterized by hyperplasia, metaplasia, dysplasia or aplasia, it was located in the nucleus, suggesting that the localization of apoptin is related to its activity (Danen-van Oorschot et al. 1997). Apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn, 1996).

Therefore, apoptin is a therapeutic compound for the selective destruction of tumor cells, or other hyperplasia, metaplasia, a- or dysplasia, especially for those tumor cells which have become resistant to (chemo)-therapeutic induction of apoptosis, due to the lack of functional p53 and (over)-expression of Bcl-2 and other apoptosis-inhibiting agents (Noteborn and Pietersen, 1998). It appears, that even pre-malignant, minimally transformed cells, are sensitive to the death-inducing effect of apoptin. In addition, Noteborn and Zhang (1998) have shown that apoptin-induced apoptosis can be used as diagnosis of cancer-prone cells and treatment of cancer-prone cells.

The fact that apoptin does not induce apoptosis in normal human cells, at least not in vitro, shows that a toxic effect of apoptin treatment in vivo will be very low. Noteborn and Pietersen (1998) and Pietersen et al. (1998) have provided evidence that adenovirus expressed apoptin does not have an acute toxic effect in vivo. In addition, in nude mice it was shown that apoptin has a strong anti-tumor activity.

However, to further enlarge the array of therapeutic anti-cancer or anti-auto-immune-disease compounds available in the art, additional therapeutic compounds are desired that are designed to work alone, sequentially to, or jointly with apoptin, especially in those cases wherein p53 is (partly) non-functional.

The invention provides novel therapeutic possibilities, for example novel combinatorial therapies or novel therapeutic compounds that can work alone, sequentially to, or jointly with apoptin, especially in those cases wherein p53 is (partly) non-functional. In a first embodiment, the invention provides an isolated or recombinant nucleic acid or functional equivalent or fragment thereof encoding an apoptin-associating proteinaceous substance capable of providing apoptosis, alone or in combination with other apoptosis inducing substances, such as apoptin. Proteinaceous substance herein is defined as a substance comprising a peptide, polypeptide or protein, optionally having been modified by for example glycosylation, myristilation, phosporylation, the addition of lipids, by homologous or heterologous di-or multimerisation, or any other (posttranslational) modifications known in the art.

Apoptin-associating herein is defined as belonging to the cascade of substances specifically involved in the cascade of events found in the apoptosis pathway as inducable by apoptin, preferably those substances that are specifically involved in the p53-independent apoptosis pathway.

In a preferred embodiment, the invention provides an isolated or recombinant nucleic acid or functional equivalent or fragment thereof encoding an apoptin-associating proteinaceous substance capable of providing apoptosis derived from a cDNA library, preferably a vertebrate cDNA library, such as derivable from poultry, but more preferably a mammalian cDNA library, preferably wherein said cDNA library comprises human cDNA.

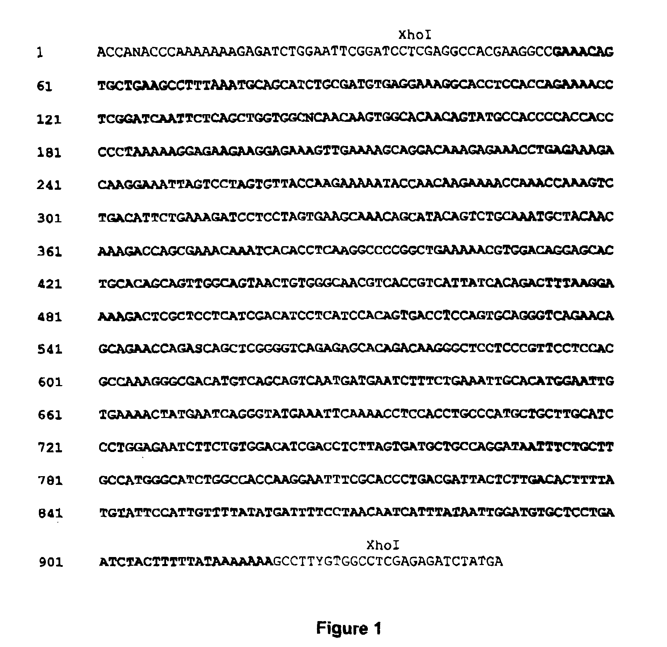

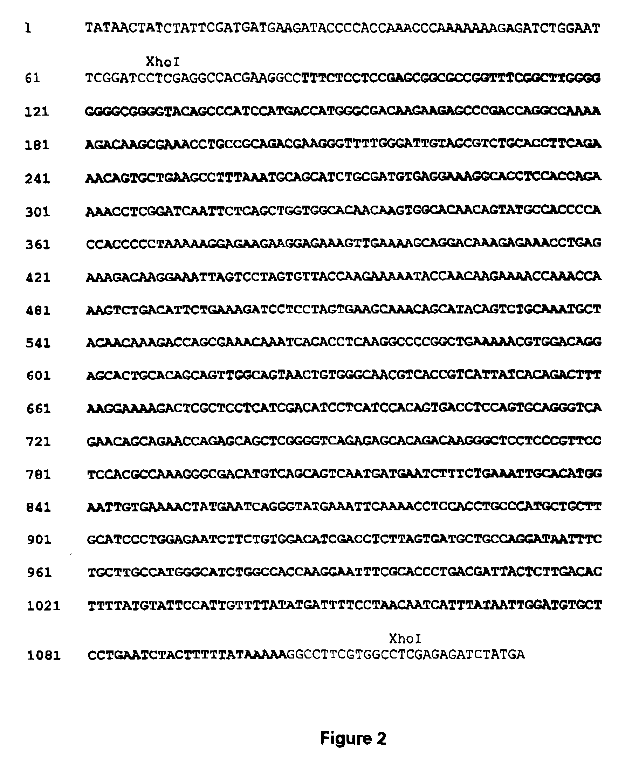

In another embodiment, the invention provides an isolated or recombinant nucleic acid or functional equivalent or fragment thereof encoding an apoptin-associating proteinaceous substance capable of providing apoptosis capable of hybridising to a nucleic acid molecule encoding an apoptin-associating proteinaceous substance capable of providing apoptosis as shown in figure 1 or 2, in particular encoding a novel protein capable of providing apoptosis or functional equivalent or functional fragment thereof called apoptin-associating protein 1, abbreviated herein also as AAP-1. Of course, an isolated or recombinant nucleic acid or functional equivalent or fragment thereof encoding an additional apoptin-associating proteinaceous substance capable of associating with the AAP-1 protein are herewith also provided, means and methods to arrive at such an additional protein located in the apoptin cascade follow those of the detailed description given herein.

In particular, the invention provides an isolated or recombinant nucleic acid or functional equivalent or fragment thereof encoding an apoptin-associating proteinaceous substance capable of providing apoptosis being at least 70% homologous, preferably at least 80%, more preferably at least 90%, most preferably at least 95% homologous to a nucleic acid molecule, or to a functional equivalent or functional fragment thereof, encoding an apoptin-associating proteinaceous substance as shown in figure 1 or 2.

Furthermore, the invention provides a vector comprising a nucleic acid according to the invention. Examples of such a vector are given in the detailed description given herein; such as vector pMT2SM-AAP-1-a or b, pMT2SM vector expressing Myc-tagged AAP-1-a or AAP-1-b cDNAs, a plasmid expressing an apoptin-associating protein fragment, and so on. These and other vectors are for example useful in finding additional apoptin-associating proteinaceous substances from the cascade, as defined above.

In yet another embodiment, the invention provides a vector comprising a nucleic acid according to the invention, said vector comprising a gene-delivery vehicle, making the invention very useful in gene therapy. By equiping a gene delivery vehicle with a nucleic acid according to the invention, and by targeting said vehicle to a cell or cells that have been over-proliferating and/or have shown decreased death rates, said gene delivery vehicle provides said cell or cells with the necessary means for apoptosis, providing far reaching therapeutic possibilities.

Furthermore, the invention provides a host cell comprising a nucleic acid or a vector according to the invention. Examples comprise transformed or transfected bacterial or yeast cells as described in the detailed description herein. Preferred is a host cell according to the invention which is a transformed eukaryotic cell such as a yeast cell or a vertebrate cell, such as mammalian or Cos cells transformed or transfected with a nucleic acid or vector according to the invention. Said cells are in general capable to express or produce a proteinaceous substance capable of providing apotosis with the ability to associate with apoptin.

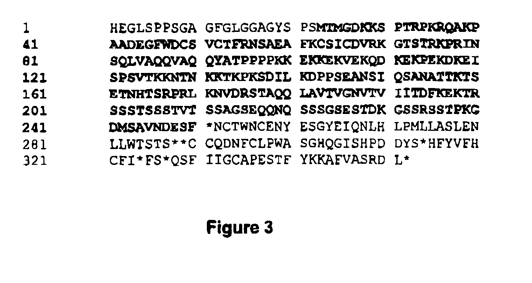

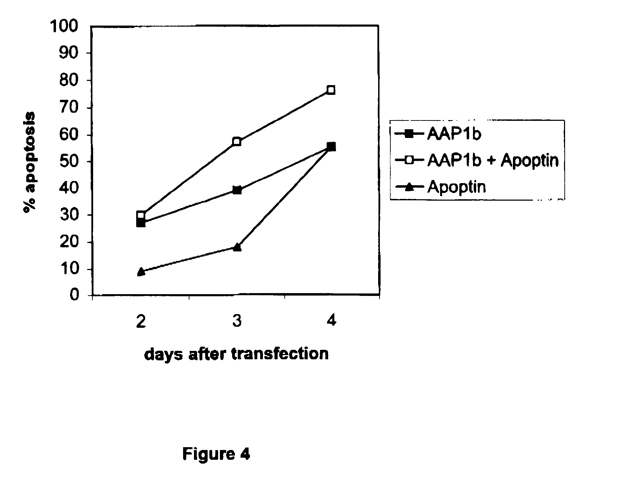

The invention furthermore provides an isolated or recombinant apoptin-associating proteinaceous substance capable of providing apoptosis. As for example shown herein in figure 4, expression of such apoptin-associating proteinaceous substance in cells, such as tumour cells, or other over-proliferating cells, induces the apoptic process. It can do so alone, or in the presence of other apoptosis inducing substances such as apopotin, and especially so independent of p53, showing that also in those cases where (functional) p53 is absent apoptosis can be induced by a substance according to the invention. In particular, the invention provides a proteinaceous substance according to the invention encoded by a nucleic acid according to the invention, for example comprising at least a part of an amino acid sequence as shown in figure 3 or a functional equivalent or functional fragment thereof capable of providing apoptosis alone or in combination with other apotosis inducing substances such as apoptin.

The invention also provides an isolated or synthetic antibody specifically recognising a proteinaceous substance or functional equivalent or functional fragment thereof according to the invention. Such an antibody is for example obtainable by immunising an experimental animal with a apoptin-associating proteinaceous substance or an immunogenic fragment or equivalent thereof and harvesting polyclonal antibodies from said immunised animal (as shown herein in the detailed description), or obtainable by other methods known in the art such as by producing monoclonal antibodies, or (single chain) antibodies or binding proteins expressed from recombinant nucleic acid derived from a nucleic acid library, for example obtainable via phage display techniques.

With such an antibody, the invention also provides a proteinaceous substance specifically recognisable by such an antibody according to the invention, for example obtainable via immunoprecipitation, Western Blotting, or other immunological techniques known in the art.

Furthermore, the invention provides use of a nucleic acid, vector, host cell, or proteinaceous substance according to the invention for the induction of apoptosis, as for example shown in figure 4. In particular, such use is provided wherein said apoptosis is p53-independent. In particular, such use is also provided further comprising use of a nucleic acid encoding apoptin or a functional equivalent or fragment thereof or use of apoptin or a functional equivalent or fragment thereof. As can be seen from figure 4, combining these apoptin-inducing substances increases the percentage apoptosis of treated tumour cells.

Such use as provided by the invention is particularly useful from a therapeutic viewpoint. The invention provides herewith a pharmaceutical composition comprising a nucleic acid, vector, host cell, or proteinaceous substance according to the invention. In addition, such a pharmaceutical composition according to the invention is provided further comprising a nucleic acid encoding apoptin or a functional equivalent or fragment thereof or apoptin or a functional equivalent or fragment thereof.

Such a pharmaceutical composition is in particular provided for the induction of apoptosis, for example wherein said apoptosis is p53-independent, for the treatment of a disease where enhanced cell proliferation or decreased cell death is observed, as is in general the case when said disease comprises cancer or auto-immune disease. Herewith the invention provides a method for treating an individual carrying a disease where enhanced cell proliferation or decreased cell death is observed comprising treating said individual with a pharmaceutical composition according to the invention. In particular these compositions comprise a factor of an apoptosis pathway, which is specific for transformed cells. Therefore, these compositions are essential for new treatments, but also for diagnosis of diseases related with aberrancies in the apoptotic process, such as cancer and auto-immune diseases.
The invention will be explained in more detail in the following detailed description which is not limiting the invention.

### Detailed description

We have used the yeast-2 hybrid system (Durfee et al., 1993) to identify apoptin-associating cellular compounds, which are essential in the induction of apoptosis. The used system is an in-vivo strategy to identify human proteins capable of physically associating with apoptin. It has been used to screen cDNA libraries for clones encoding proteins capable of binding to a protein of interest (Fields and Song, 1989, Yang et al., 1992). The invention provides a for example novel apoptin-associating protein, one of which is named apoptin-associating protein 1, abbreviated as AAP-1. The invention also provides a method for inducing apoptosis through interference with the function of this newly discovered AAP-1 protein or functional equivalents or fragments thereof and/or the induction of apoptosis by means of (over)expression of AAP-1 or related gene or functional equivalents or fragments thereof.

The invention also provides an anti-tumor therapy based on the interference with the function of AAP-1-like proteins and/or its (over)expression. AAP-1-like proteins are normally not very abundantly present in immortalized cell lines. Therefore, an aberrant high level of AAP-1-like proteins will result in the induction of the opposite process of cell transformation, namely apoptosis. The invention furthermore provides the mediator of apoptin-induced apoptosis, which is tumor-specific. The invention provides a therapy for cancer, auto-immune diseases or related diseases which is based on AAP-1-like proteins alone or in combination with apoptin and/or apoptin-like compounds.

### Construction of pGBT9-VP3

For the construction of the bait plasmid, which enables the identification of apoptin-associating proteins by means of a yeast-two-hybrid system, plasmid pET-16b-VP3 (Noteborn, unpublished results) was treated with NdeI and BamHI. The 0.4 kb NdeI-BamHI DNA fragment was isolated from low-melting-point agarose. Plasmid pGBT9 (Clontech Laboratories, Inc, Palo Alto, USA) was treated with the restriction enzymes EcoRI and BamHI. The about 5.4-kb DNA fragment was isolated and ligated to an EcoRI-NdeI linker and the 0.4-kb DNA fragment containing the apoptin-encoding sequences starting from its own ATG-initiation codon. The final construct containing a fusion gene of the GAL4-binding domain sequence and apoptin under the regulation of the yeast promoter ADH was called pGBT-VP3 and was proven to be correct by restriction-enzyme analysis and DNA-sequencing according to the Sanger method (1977).

All cloning steps were essentially carried out as described by Maniatis et al. (1992). The plasmid pGBT-VP3 was prurified by centrifugation in a CsCl gradient and column chromatography in Sephacryl S500 (Pharmacia).

### GAL4-activation domain-tagged cDNA library

The expression vector pACT, containing the cDNAs from Epstein-Barr-virus-transformed human B cells fused to the GAL4 transcriptional activation domain, was used for detecting apoptin-associating proteins. The pACT cDNA lbrary is derived from the lambda-ACT cDNA library, as described by Durfee et al. 1993.

### Bacterial and Yeast strains

The E.coli strain JM109 was the transformation recipient for the plasmid pGBT9 and pGBT-VP3. The bacterial strain electromax/DH10B was used for the transformation needed for the recovery of the apoptin-associating pACT-cDNAs, and was obtained from GIBCO-BRL, USA.

The yeast strain Y190 was used for screening the cDNA library, and all other transformations, which are part of the used yeast-two-hybrid system.

### Media

For drug selections Luria Broth (LB) plates for E.coli were supplemented with ampicillin (50 microgram per ml). Yeast YPD and SC media were prepared as described by Rose et al. (1990).

Transformation of competent yeast strain Y190 with plasmids pGBT-VP3 and pACT-cDNA and screening for beta-galactosidase activity.

The yeast strain Y190 was made competent and transformed according to the methods described by Klebe et al. (1983). The yeast cells were first transformed with pGBT-VP3 and subsequently transformed with pACT-cDNA, and these transformed yeast cells were grown on histidine-minus plates, also lacking leucine and tryptophan.

Hybond-N filters were layed on yeast colonies, which were histidine-positive and allowed to wet completely. The filters were lifted and submerged in liquid nitrogen to permeabilize the yeast cells. The filters were thawed and layed with the colony side up on Whattman 3MM paper in a petridish with Z-buffer (Per liter: 16.1 gr Na₂HPO₄.7H₂O, 5.5 gr NaH₂PO₄.H₂O, 0.75 gr KCl and 0,246 gr MgSO₄.7H₂O, pH 7.0) containing 0.27% beta-mercapto-ethanol and 1 mg/ml X-gal. The filters were incubated for at least 15 minutes or during night.

### Recovery of plasmids from yeast

Total DNA from yeast cells, which were histidine- and beta-galactosidase-positive, was prepared by using the glusulase-alkaline lysis method as described by Hoffman and Winston (1987) and used to transform Electromax/DH10B bacteria via electroporation using a Bio-Rad GenePulser according the manufacturer's specifications.

Transformants were plated on LB media containing the antibiotic agent ampicillin.

### Isolation of apoptin-associating pACT clones

By means of colony-filter assay the colonies were lysed and hybridized to a radioactive-labeled 17-mer oligomer, which is specific for pACT (see also section Sequence analysis). Plasmid DNA was isolated from the pACT-clones, and by means of XhoI digestion analysed for the presence of a cDNA insert.

### Sequence analysis

The subclones containing the sequences encoding apoptin-associating proteins were sequenced using dideoxy NTPs according to the Sanger-method, which was performed by Eurogentec, Seraing, Belgium). The used sequencing primer was a pACT-specific 17-mer comprising of the DNA-sequence 5'-TACCACTACAATGGATG-3'.

The sequences of the apoptin-associating cDNAs were compared with known gene sequences from the EMBL/Genbank.

### Results and discussion

Apoptin induces specifically apoptosis in transformed cells, such as cell lines derived from human tumors. To identify the essential compounds in this cell-transformation-specific and/or tumor-specific apoptosis pathway, a yeast genetic screen was carried out. We have used a human cDNA library, which is based on the plasmid vector pACT containing the complete cDNA copies made from Epstein-Barr virus-transformed human B cells (Durfee et al., 1993).

Construction of a bait plasmid expressing a fusion gene product of GAL4-DNA-binding domain and apoptin

To examine the existence of apoptin-associating proteins in the human transformed/tumorigenic cDNA library, a so-called bait plasmid had to be constructed. To that end, the complete apoptin-encoding region, flanked by about 40 basepairs downstream from the apoptin gene, was cloned in the multiple cloning site of plasmid pGBT9.

The final construct, called pGBT-VP3, was analysed by restriction-enzyme analysis and sequencing of the fusion area between apoptin and the GAL4-DNA-binding domain.

A gene(fragment) encoding an apoptin-associating protein is determined by transactivation of a GAL4-responsive promoter in yeast.

The apoptin gene is fused to the GAL4-DNA-binding domain of plasmid pGBT-VP3, whereas all cDNAs derived from the transformed human B cells are fused to the GAL4-activation domain of plasmid pACT. If one of the proteinaceous substances encoded by said cDNAs binds to apoptin, the GAL4-DNA-binding domain will be in the vicinity of the GAL4-activation domain resulting in the activation of the GAL4-responsive promoter, which regulates the reporter genes HIS3 and LacZ.

The yeast clones containing plasmid expressing apoptin and a plasmid expressing an apoptin-associating protein fragment can grow on a histidine-minus medium and will stain blue in a beta-galactosidase assay. Subsequently, the plasmid with the cDNA insert encoding the apoptin-associating protein can be isolated and characterized.

Before we could do so, however, we have determined that transformation of yeast cells with pGBT-VP3 plasmid alone, or in combination with an empty pACT vector, did not result in the activation of the GAL4-responsive promoter.

### Identification of apoptin-associating proteins encoded by cDNAs derived from a human transformed B cell line.

We have found two yeast colonies, which upon transformation with pGBT-VP3 and pACT-cDNA was able to grow on a histidine-minus medium (also lacking leucine and tryptophan) and stained blue in a beta-galactosidase assay. These results indicate that the observed yeast colonies contain besides the bait plasmid pGBT-VP3 also a pACT plasmid encoding a potential apoptin-associating protein.

Plasmid DNA was isolated from the positive yeast colony, which was transformed in bacteria. By means of a filter-hybridization assay using a pACT-specific labeled DNA-probe, the clones containing pACT plasmid could be determined. Subsequently, pACT DNA was isolated and digested with restriction enzyme XhoI, which is indicative for the presence of a cDNA insert. Finally, the pACT plasmids containing a cDNA insert was sequenced by using the Sanger method (Sanger et al., 1977).

### Description of apoptin-associating proteins

The yeast genetic screen for apoptin-associating proteins resulted in the detection of two cDNA clones A and B comprising a single type of protein, namely a novel protein called apoptin-associating protein 1, abbreviated as AAP-1. The cDNA AAP-1-b harbors the complete open reading frame with an ATG-initiation codon, whereas the AAP-1-a cDNA sequence contains a partial AAP-1 open reading frame, which is completely homologous to the AAP-1-b DNA sequence.

The determined DNA sequence of the AAP-1-a and AAP-1-b cDNA clones are shown in Figures 1 and 2, respectively. The amino acid sequence, derived from the detected DNA sequence of clone AAP-1-b, which represents the complete AAP-1 a.a. sequence, is given in Figure 3.

### Construction of an expression vector for the identification of AAP-1 protein in mammalian cells.

To study whether the cloned cDNAs AAP-1-a and AAP-1-b indeed encode (apoptin-associating) protein products, we have carried out the following experiments.

The DNA plasmid pMT2SM contains the adenovirus 5 major late promoter (MLP) and the SV40 ori enabling high levels of expression of foreign genes in transformed mammalian cells, such as SV-40-transformed Cos cells. Furthermore, the pMT2SM vector contains a Myc-tag (amino acids: EQKLISEEDL) which is in frame with the foreign-gene product. This Myc-tag enables the recognition of e.g. apoptin-associating proteins by means of the Myc-tag-specific 9E10 antibody.

The pMT2SM vectors expressing Myc-tagged AAP-1-a or AAP-1-b cDNAs were constructed as follows. The pACT-AAP-1-a and pACT-AAP-1-b cDNA clones were digested with the restriction enzyme XhoI and the cDNA inserts were isolated. The expression vector pMT2SM was digested with XhoI and treated with calf intestine alkline phosphatase and ligated to the isolated AAP-1 cDNA inserts. By sequence analysis, the pMT2SM constructs containing the AAP-1-a or AAP-1-b cDNA in the correct orientation were identified.

The synthesis of Myc-tagged AAP-1 protein was analyzed by transfection of Cos cells with plasmid pMT2SM-AAP-1-a or pMT2SM-AAP-1-b. As negative control, Cos cells were mock-transfected. Two days after transfection, the cells were lysed and Western-blot analysis was carried out using the Myc-tag-specific antibody 9E10.

The Cos cells transfected with pMT2SM-AAP-1-a and pMT2SM-AAP-1-b were proven to synthesize a specific Myc-tagged AAP-1 product with the expected size of approximately 33 kDa (AAP-1-a) or 35 kDA (AAP-1-b). As expected, the lysates of the mock-transfected Cos cells did not contain a protein product reacting with the Myc-tag-specific antibodies.

These results indicate that we have been able to isolate cDNAs that are able to produce a protein product with the ability to associate to the apoptosis-inducing protein apoptin.

### Co-immunoprecipitation of Myc-tagged AAP-1 protein with apoptin in a transformed mammalian cell system.

Next, we have analysed the association of apoptin and the AAP-1 protein by means of co-immunoprecipitations using the Myc-tag-specific antibody 9E10. The 9E10 antibodies were shown not to bind directly to apoptin, which enables the use of 9E10 for carrying out co-immunoprecipitations with (myc-tagged) apoptin-associating proteins and apoptin.

To that end, Cos cells were co-transfected with plasmid pCMV-VP3 encoding apoptin and with plasmid pMT2SM-AAP-1-a. As a negative control, cells were transfected with pCMV-VP3 expressing apoptin and a plasmid pcDNA3.1.LacZ-myc/His -LacZ encoding the myc-tagged beta-galactosidase, which does not associate with apoptin.

Two days after transfection, the cells were lysed in a buffer consisting of 50 mM Tris (7.5), 250 mM NaCl, 5 mM EDTA, 0.1 % Triton X100, 1 mg/ml Na₄P₂O₇ and freshly added protease inhibitors such as PMSF, Trypsine-inhibitor, Leupeptine and Na₃VO₄. The specific proteins were immuno-precipitated as described by Noteborn et al. (1998) using the Myc-tag-specific antibodies 9E10, and analyzed by Western blotting.

Staining of the Western blot with 9E10 antibodies and 111.3 antibodies, which are specifically directed against myc-tag and apoptin,respectively, showed that the "total" cell lysates contained apoptin and the Myc-tagged AAP-1 protein or beta-galactosidase product. Immunoprecipitation of the Myc-tagged AAP-1 products was accompanied by the immuno-precipatation of apoptin product of 16 kDa. In contrast, immunoprecipitation of myc-tagged beta-galactosidase did not result in a significant co-precipitation of the Apoptin protein.

In total, three independent immunoprecipitation experiments were carried out, which all showed the associating ability of apoptin to the AAP-1 protein.

These results indicate that the novel determined AAP-1 protein is able to specifically associate with apoptin not only in the yeast background, but also in a mammalian transformed cellular system.

### Over-expression of the novel AAP-1 protein in human transformed cells induces the apoptotic process.

In addition, we have examined whether AAP-1 carries apoptotic activity. First, we have analysed the cellular localisation of the novel AAP-1 protein in human transformed cells. To that end, the human osteosarcoma-derived Saos-2 cells were transfected, as described by Danen-van Oorschot (1997), with plasmid pMT2SM-AAP-1-a or pMT2SM-AAP-1-b encoding the myc-tagged AAP-1-a or AAP-1-b protein, respectively.

By indirect immunofluorescence using the myc-tag-specific antibody 9E10 and DAPI, which stains the nuclear DNA, it was shown that both the partial and complete AAP-1 protein were present in the nucleus of the cell. Actually, it co-localizes with the chromatin/DNA structures.

Finally, we examined whether (over)-expression of both cDNAs encoding complete or partial AAP-1 protein results in induction of apoptosis. Four days after transfection, the majority of AAP-1-positive cells were aberrantly stained with DAPI, which is indicative for induction of apoptosis (Telford, 1992, Danen-van Oorschot, 1997).

Co-expression of apoptin and both AAP-1 proteins in human tumor cells, such as Saos-2 cells, results in a faster apoptotic process as expression of apoptin or AAP-1 protein alone. The results of the apoptotic activity of the complete AAP-1 protein are shown in Figure 4. The fact that AAP-1 protein can induce apoptosis in p53-minus Saos-2 cells indicates that AAP-1 can induce p53-independent apoptosis. These results imply that AAP-1 can be used as anti-tumor agent in cases where other (chemo)therapeutic agents will fail. Furthermore, the finding that both apoptin and AAP-1 induce a p53-independent pathway indicates that AAP-1 fits in the apoptin-induced apoptotic pathway.

In conclusion, we have identified an apoptin-associating protein, namely the novel AAP-1 protein, which is present in the nucleus and able to induce (p53-independent) apoptosis in human tumor cells.

### Production of polyclonal antibodies directed against AAP-1 proteins.

For the production of polyclonal antibodies against AAP-1 proteins a putative immunogenic peptide was synthesized (AAP-1 peptide consists of the amino acids N/terminus-CTKTSETNHTSRPRLK-C/terminus; EuroGentec SA, Belgium). Subsequently, rabbits were injected with the specific peptides according the standard procedures of the manufacturer.

The serum derived from the rabbits injected with the AAP-1 peptide was shown to be specific for the above described AAP-1 products by means of ELISA and Western-blot assays. These results imply that we have generated specific antibodies, which can be used for detecting the Apoptin-associating protein AAP-1.

In conclusion, we have provided evidence that interference of specific factors with the function of AAP-1 proteins results in induction of apoptosis. Therapies based on induction of (p53-independent) apoptosis are possible utilising the interference with the function of AAP-1 proteins. An example of such an interfering factor is apoptin. Another CAV-derived protein, which is known to induce apoptosis and also known to enhance apoptin activity is VP2 (Noteborn et al., 1997).

### Description of the figures

Figure 1 shows the partial sequence of vector pMT2SM-AAP-1-a. The DNA sequence of the AAP-1-a cDNA is given in bold.

Figure 2 shows the partial sequence of vector pMT2SM-AAP-1-b. The DNA sequence of the AAP-1-b cDNA is given in bold.

Figure 3 shows the amino-acid sequence of the analysed region of the apoptin-associating clone AAP-1-b (bold). In addition, the three C-terminal amino acids H-E-G of the multiple cloning site of pACT are given to illustrate that the AAP-1 amino acid sequence is in frame with the GAL4-activation domain. This feature proves that the AAP-1 region is indeed synthesised in yeast cells. Note that in figure 3 amino acid position 23 corresponds with the first amino acid of an AAP-1 like protein. Functional domains or fragments herein can for example be identified as a transcription factor binding domain running from amino acid position 1 ( = 23 in figure 3) to about 54; a zinc-finger motive, protein-protein interaction and/or protein-nucleic acid interaction domain running from about amino acid position 25 ( = 47 in figure 3) to about 42; an apoptosis associated region running from about amino acid position 32 to 226; a nuclear localisation signal running from about amino acid position 74 to 81; and a nuclear localisation signal running from about amino acid position 102 to 108, or at equivalent positions in another AAP-1 like protein.

Figure 4 shows the apoptotic activity of AAP-1-b protein in Saos-2 cells, when expressed alone (filled square) or in combination with apoptin (open square). The percentage of apoptin-induced apoptosis is also indicated (filled triangle)

### REFERENCES

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
3. Danen-Van Oorschot, A.A.A.M, Den Hollander, A., Takayama, S., Reed, J., Van der Eb, A.J. and Noteborn, M.H.M. (1997a). BAG-1 inhibits p53-induced but not apoptin-induced apoptosis. Apoptosis 2, 395-402.
4. Duke, R.C., Ocjius, D.M., Young, J, D-E. (1996). Cell suicide in health and disease. Scientific American December 1996, 48-55.
5. Durfee, T., Becherer, K., Chen, P.-L., Yeh,S.-H., Yang, Y., Kilburn, A.E., Lee, W.-H., and Elledge, S.J. (1993). The retinoblastoma protein associates with the protein phosphate type I catalytic subunit. Genes and Development 7, 555-569.
6. Earnshaw, W.C., 1995. Nuclear changes in apoptosis. Current Opinion in Cell Biology 7, 337-343.
7. Fields, S. and Song, O.K. (1989). A novel genetic system to detect protein-protein interactions. Nature 340, 245-246.
8. Hockenberry, D.M. (1994). Bcl-2 in cancer, development and apoptosis. Journal of Cell Science, Supplement 18, 51-55.
9. Hoffman, C.S. and Winston, F. (1987). A ten-minute DNA preparation from yeast efficiently releases autonomous plasmids for transformation of Escherichia coili. Gene 57, 267-272.
10. Kerr, J.F.R., Winterford, C.M., and Harmon, B.V. (1994). Apoptosis: Its significance in cancer and cancer therapy. Cancer 73, 2013-2026.
11. Klebe, R.J., Harriss, J.V., Sharp, Z.D., and Douglas, M.G. (1983). A general method for polyethylene-glycol-induced genetic transformation of bacteria and yeast. Gene 25, 333-341.
12. Levine, A.J. (1997). p53, the cellular gatekeeper for growth and division. Cell 88, 323-331.
13. Maniatis, T., Fritsch, E.F., and Sambrook, J. (1982). Molecular Cloning: A Laboratory Manual. CSHL Press, New York, USA.
14. McDonell T.J., Meyn, R.E., Robertson, L.E. (1995). Implications of apoptotic cell death regulation in cancer therapy. Seminars in Cancer Biology 6, 53-60.
15. Noteborn, M.H.M. (1996). PCT application WO 96/41191. Apoptin induces apoptosis in human transformed and malignant cells but not in normal cells as essential characteristic for the development of an anti-tumor therapy.
16. Noteborn, M.H.M., and De Boer, G.F. (1996). Patent USA/no. 030, 335.
17. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
18. Noteborn, M.H.M., and Pietersen, A. (1998). A gene delivery vehicle expressing the apoptosis-inducing proteins VP2 and/or apoptin. PCT Application no. PCT/NL98/00213
19. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
20. Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology, 79, 3073-3077.
21. Noteborn, M.H.M., and Zhang, Y. (1998). Methods and means for determining the transforming capability of agents, for determining the predisposition of cells to become transformed and prophylactic treatment of cancer using apoptin-like activity. PCT Application no. PCT/NL98/00457
22. Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
23. Paulovich, A.G., Toczyski, D., Hartwell, H. (1997). When checkpoints fail. Cell 88, 315-321.
24 Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1998). Specific tumor-cell killing with adenovirus vectors containing the apoptin gene. Gene Therapy 6, 882-892.
25. Rose, M.D., Winston, F., and Hieter, P. (1990). Methods in yeast genetics. A laboratory course manual. Cold Spring Harbor Laboratory Press, Cold Spring Harbor, New York, USA.
26. Sachs, L. and Lotem, J. (1993). Control of programmed cell death in normal and leukemia cells: New implications for therapy. Blood 82, 15-21.
27. Sanger, F., Nicklen, S., and Coulsen, A.R. (1977). DNA sequencing with chain-terminating inhibitors. Proceedings National Academic Sciences USA 74, 5463-5467.
28. Steller, H. (1995). Mechanisms and genes of cellular suicide. Science 267, 1445-1449.
29. Telford, W.G., King, L.E., Fraker, P.J. (1992). Comparative evaluation of several DNA binding dyes in the detection of apoptosis-associated chromatin degradation by flow cytometry. Cytometry 13, 137-143.
30. Teodoro, J.G. and Branton, P.E. (1997). Regulation of apoptosis by viral gene products. Journal of Virology 71, 1739-1746.
31. Thompson, C.B. (1995). Apoptosis in the pathogenesis and treatment of disease. Science 267, 1456-1462.
32. White, E. (1996). Life, death, and the pursuit of apoptosis. Genes and development 10, 1-15.
33. Wyllie, A.H. (1995). The genetic regulation of apoptosis. Current Opinion in Genetics and Development 5, 97-104.
34. Wyllie, A.H., Kerr, J.F.R., Currie, A.R. (1980). Cell death: The significance of apoptosis. International Review of Cytology 68, 251-306.
35. Yang, X., Hubbard, E.J.A., and Carlson, M. (1992). A protein kinase substrate identified by the two-hybrid system. Science 257, 680-682.
36. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 S1, 118-120.
37. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

## Claims

1. An isolated or recombinant nucleic acid or functional equivalent or functional fragment thereof encoding an apoptin-associating proteinaceous substance capable of providing apoptosis.

2. A nucleic acid according to claim 1 derived from a cDNA library.

3. A nucleic acid according to claim 1 or 2 wherein said cDNA library comprises human cDNA.

4. A nucleic acid according to anyone of claims 1 to 3 capable of hybridising to a nucleic acid molecule encoding an apoptin-associating proteinaceous substance as shown in figure 1 or 2.

5. A nucleic acid according to anyone of claims 1 to 4 being at least 70% homologous to a nucleic acid molecule, or to a functional equivalent or functional fragment thereof, encoding an apoptin-associating proteinaceous substance as shown in figure 1 or 2.

6. A vector comprising a nucleic acid according to anyone of claims 1 to 5.

7. A vector according to claim 6 comprising a gene-delivery vehicle.

8. A host cell comprising a nucleic acid according to anyone of claims 1 to 5 or a vector according to claim 6 or 7.

9. A host cell according to claim 8 which is a eukaryotic cell such as a yeast cell or a vertebrate cell.

10. An isolated or recombinant apoptin-associating proteinaceous substance capable of providing apoptosis.

11. A proteinaceous substance according to claim 10 encoded by a nucleic acid according to anyone of claims 1 to 5.

12. A proteinaceous substance according to claim 10 or 11 comprising at least a part of an amino acid sequence as shown in figure 3 or a functional equivalent or functional fragment thereof.

13. An isolated or synthetic antibody specifically recognising a proteinaceous substance or functional equivalent or functional fragment thereof according to anyone of claims 10 to 12.

14. A proteinaceous substance specifically recognisable by an antibody according to claim 13.

15. Use of a nucleic acid according to anyone of claims 1 to 5, a vector according to claims 6 or 7, a host cell according to claim 8 or 9, a proteinaceous substance according to anyone of claims 10 to 12 or 14 for the induction of apoptosis.

16. Use according to claim 15 wherein said apoptosis is p53-independent.

17. Use according to claim 15 or 16 further comprising use of a nucleic acid encoding apoptin or a functional equivalent or fragment thereof or use of apoptin or a functional equivalent or fragment thereof.

18. A pharmaceutical composition comprising a nucleic acid according to anyone of claims 1 to 5, a vector according to claims 6 or 7, a host cell according to claim 8 or 9, a proteinaceous substance according to anyone of claims 10 to 12 or 14.

19. A pharmaceutical composition according to claim 18 further comprising a nucleic acid encoding apoptin or a functional equivalent or fragment thereof or apoptin or a functional equivalent or fragment thereof.

20. A pharmaceutical composition according to claim 18 or 19 for the induction of apoptosis.

21. A pharmaceutical composition according to claim 20 wherein said apoptosis is p53-independent.

22. A pharmaceutical composition according to anyone of claims 18 to 21 for the treatment of a disease where enhanced cell proliferation or decreased cell death is observed.

23. A pharmaceutical composition according to claim 22 wherein said disease comprises cancer or auto-immune disease.

24. A method for treating an individual carrying a disease where enhanced cell proliferation or decreased cell death is observed comprising treating said individual with a pharmaceutical composition according to anyone of claims 18 to 23.
